# EUROPEAN PATENT SPECIFICATION

(11) **EP 1 001 720 B1**
(45) Date of publication and mention of the grant of the patent: **02.10.2002**
(21) Application number: 98933252.3
(22) Date of filing: 08.07.1998
(51) Int. Cl.: A61F 2/14

(54) **INTRACORNEAL DIFFRACTIVE LENS**
INTRAKORNEALE DIFFRAKTIVE LINSE
LENTILLE INTRA-CORNEENNE DIFFRACTIVE

(30) Priority: 07.08.1997 US 908230
(43) Date of publication of application: 24.05.2000
(73) Proprietor: ALCON LABORATORIES, INC., Fort Worth Texas 76134-2099 (US)
(72) Inventor: PATEL, Anilbhai, S., Arlington, TX 76017 (US); LeBOEUF, Albert, R., Forth Worth, TX 76133 (US)
(74) Representative: Hanna, Peter William Derek
(86) International application number: US9814119
(87) International publication number: WO99007309

(56) References cited:
- EP-A- 0 335 731
- EP-A- 0 382 620
- EP-A- 0 420 549

## Description

### Background of the Invention

This invention relates generally to the field of optical intracorneal lenses (ICL) and, more particularly, to diffractive optic ICLs.

The human eye in its simplest terms functions to provide vision by transmitting light through a clear outer portion called the cornea, and focusing the image by way of a crystalline lens onto a retina. The quality of the focused image depends on many factors including the size and shape of the eye, and the transparency of the cornea and the lens.

The optical power of the eye is determined by the optical power of the cornea and the crystalline lens. In the normal, healthy eye, sharp images are formed on the retina (emmetropia). In many eyes, images are either formed in front of the retina because the eye is abnormally long (axial myopia), or formed in back of the retina because the eye is abnormally short (axial hyperopia). The cornea also may be asymmetric or toric, resulting in an uncompensated cylindrical refractive error referred to as corneal astigmatism. In addition, due to age-related reduction in lens accommodation, the eye may become presbyopic resulting in the need for a bifocal or multifocal correction device.

In the past, axial myopia, axial hyperopia and corneal astigmatism generally have been corrected by spectacles or contact lenses, but there are several refractive surgical procedures that have been investigated and used since 1949. Barraquer investigated a procedure called keratomileusis that reshaped the cornea using a microkeratome and a cryolathe. This procedure was never widely accepted by surgeons. Another procedure that has gained widespread acceptance is radial and/or transverse incisional keratotomy (RK or AK, respectively). Recently, the use of photablative lasers to reshape the surface of the cornea (photorefractive keratectomy or PRK) or for mid-stromal photoablation (Laser-Assisted In Situ Keratomileusis or LASIK) have been approved by regulatory authorities in the U.S. and other countries. All of these refractive surgical procedures cause an irreversible modification to the shape of the cornea in order to effect refractive changes, and if the correct refraction is not achieved by the first procedure, a second procedure or enhancement must be performed. Additionally, the long-term stability of the correction is variable because of the variability of the biological wound healing response between patients.

Permanent intracorneal implants made from synthetic materials are also known for the correction of corneal refractive errors. For example, U.S. Patent No. 5,123,921 (Werblin, et al.) discloses an intracomeal lens that is implanted intrastromally using a microkeratome. The lens itself has little refractive power, but changes the refractive power of the cornea by modifying the shape of the anterior surface of the cornea. The microkeratome used to implant this lens is complex and expensive and the lens requires a great deal of surgical skill to implant.

Keravision owns a series of patents related to an intrastromal ring device used to induce refractive changes in the cornea (see U.S. Patent Nos. 5,505,722, 5,466,260, 5,405,384, 5,323,788, 5,318,047, 5312,424, 5,300,118, 5,188,125, 4,766,895, 4,671,276 and 4,452,235). The use of a ring-shaped device avoids implantation of the device within the central optical zone of the cornea, and is implanted in peripheral groove made by a special surgical instrument. The ring itself has no refractive power. Refractive changes are caused by the implanted ring changing the shape of the anterior surface of the cornea.

A variation of the intrastromal ring is called Gel Injection Adjustable Keratoplasty (GIAK) and is described in U.S. Patent Nos. 5,090,955 (Simon), 5,372,580 (Simon, et al.) and WIPO Publication No. WO 96/06584. Instead of a solid device, these publications disclose injecting a ring of biocompatible gel around the optic zone of the stroma to effect refractive changes to the cornea by changing the shape of the cornea.

These prior art intracorneal devices all work by changing the shape of the cornea, and the devices themselves have little or no refractive properties. As a result, the preparation of the lamellar bed into which these devices are inserted is critical to the predictability of the refractive outcome, requiring very precise microkeratomes or other special surgical instruments and a great deal of surgical skill for success.

Various intracomeal implants having a refractive power are also known. For example, U.S. Patent No. 4,607,617 (Choyce) describes an implant made of polysulfone (refractive index 1.633). The high refractive index of polysulfone relative to stromal tissue (1.375) results in an implant that acts as an optical lens that effects a refractive change to the cornea without relying on a change in corneal shape. This lens was never clinically or commercially acceptable because the polysulfone material is too impermeable to glucose and other metabolites to maintain the corneal tissue anterior to the implant. Corneal ulcerations, opacifications and other complications were the clinical result.

An implant that attempts to overcome the complications of polysulfone implants is described in U.S. Patent No. 4,624,669 (Grendahl). This implant contains a plurality of microfenestrations that allows the flow of glucose and other metabolites through the lens. In animal studies, however, the microfenestrations were filled with keratocytes that created opacities, resulting in unacceptable light scattering and visual acuities. As a result, this implant was never commercially developed. In an attempt to limit damage to the anterior cornea and prevent keratocyte ingrowth, U.S. Patent No. 5,628,794 (Lindstrom) discloses a limited diameter (2.5 mm) refractive multifocal implant for correction of presbyopia made from a rigid material having fenestrations, the implant and the fenestrations being coated with a hydrogel material. The inventors are not aware of clinical data for this lens. This limited diameter multifocal lens is not clinically acceptable for monofocal correction of myopia or hyperopia in most patients with normal pupil size under normal environmental light conditions.

Previous attempts to correct presbyopic vision have generally been limited to spectacles or contact lenses. Recently, clinical investigations were initiated for a limited diameter (less than 2.5 mm), low water content (approximately 45%) monofocal hydrogel inlay that effectively created a multifocal cornea. These early clinical investigations; however, have not been encouraging due to compromised distance vision and unacceptable multifocal vision. These lens are described in U.S. Patent Nos. 5,196,026 and 5,336,261 (Barren, et al.)

Despite these prior attempts to make a suitable comeal implant, a need continues to exist for a safe and biocompatible intracorneal lens that does not require implantation via a microkeratome and does not rely on induced shape changes to the cornea to correct refractive errors of the eye.

A diffractive intracorneal lens comprising hydrogel materials is described in EP-A-0,420,549 (Kingston Technologies) listing numerous polymers which may be used. The present invention is based on a selection of hydrogel materials denominated in the art.

### Brief Summary of the Invention

The present invention improves upon the prior art by providing a diffractive optical ICL in accordance with claims which follow, made from two different hydrogel materials that are biologically acceptable for long term implantation in the cornea. The first material contains a diffractive surface and has a higher refractive index than the cornea. The second material is bound to the first material to cover the diffractive surface and has a refractive index similar to corneal tissue. The adequate permeability of metabolites through both of the hydrogels of the diffractive ICL yields a safe implant for the cornea.

Accordingly, one objective of the present invention is to provide a safe and biocompatible intracomeal lens.

Another objective of the present invention is to provide a safe and biocompatible intracomeal lens with a high optical power.

Still another objective of the present invention is to provide a safe and biocompatible intracomeal lenses that does not rely on induced shape changes to the cornea to correct refractive errors of the eye.

Still another objective of the present invention is to provide a safe and biocompatible intracorneal lenses that contains a diffractive surface.

Still another objective of the present invention is to provide aₒ safe and biocompatible intracorneal lenses that prevents unacceptable cellular ingrowth and deposits.

These and other advantages and objectives of the present invention will become apparent from the detailed description and claims that follow.

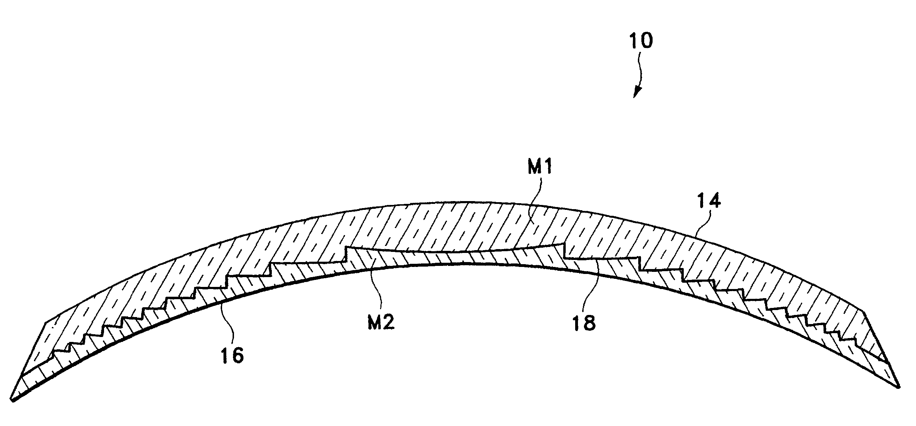

### Brief Description of the Drawing

The drawing is a cross-section view of the ICL of the present invention.

### Detailed Description of the Invention

ICL 10 of the present invention is designed to be implanted within a cornea and generally includes base lens 14 having a diffractive surface 18, that is covered by coating 16. Base lens 14 preferably has a diameter of at least 5 millimeters. Base lens 14 is preferably made from a material ("M1") which has a relatively high equilibrium water content at approximately body temperature, preferably 50% or greater, with a refractive index greater than corneal tissue and greater than 1.40. A high water content helps to ensure the flow of glucose and other metabolites through base lens 14. A high refractive index material M1 in combination with diffractive surface 18 allows ICL 10 to be made relatively thin but still have its own refractive power. While it is desirable for the material used to make base lens 14 to have as high of a water content and a refractive index as possible, increasing the water content of any high refractive index material will necessarily decrease the refractive index of that material because of the relatively low refractive index of water (1.336). In order to effect the desired refractive change to the cornea while maintaining an overall thin lens (less than 150 microns being preferred and 50 microns to 100 microns being most preferred) diffractive surface 18 is formed on base lens 14. Diffractive surface 18 increases the power of ICL 10 without increasing the overall thickness of ICL 10. The construction of diffractive surface 18 is well-known in the art and is described in U.S. Patent Nos. 5,129,718 (Futhey, et al.), U.S. Patent Nos. 5,076,684 and 5,116,111 (Simpson, et al.), U.S. Patent Nos. 4,162,122, 4,210,391, 4,338,005, 4,340,283, 4,995,714, 4,995,715, 4,881,804, 4,881,805, 5,017,000, 5, 054, 905, 5,056,908, 5,120,120, 5,121,979, 5,121,980, 5,144,483, 5,117,306 (Cohen) and U.S. Patent Nos. 4,637,697, 4,641,934 and 4,655,565 (Freeman), It will be understood to those skilled in the art that ICL 10 may be constructed to correct myopia, hyperopia, presbyopia and/or astigmatism by using diffractive monofocal or multifocal optics and superimposing or blending refractive optics when needed to correct astigmatism.

Any of a variety of hydrogel materials having the correct physical properties may be used as M1 to form base lens 14. M1 must have sufficient mechanical strength to allow for folding or rolling of ICL 10; M1 must be photo stable; and M1 preferably already has been shown safe in the contact lens and/or intraocular lens industry. Suitable monomers for M1 include aryl methacrylates, arylalkyl (meth)acrylates, naphthyl (meth)acrylates, styrene, methylstyrene, N-vinylcarbazole, N,N dimethylacrylamides, 2-phenylethyl methacrylate, 3-phenylpropyl methacrylate, 4-phenylbutyl methacrylate, 2-phenoxyethyl methacrylate, 3-phenoxypropyl methacrylate, 4-phenoxybutyl methacrylate, beta naphthyl methacrylate, N-vinylcarbazole, N-vinylpyrrolidone, hydroxyethyl (meth)acrylates, polyethylene glycol (meth)acrylates, polyethylene oxide (meth)acrylates, 3-methoxy-2-hydroxypropyl-(meth)acrylate, (meth)acrylic acid and dihydroxyalkyl (meth)acrylates.
One preferred formulation for M1 is:
N-vinyl-pyrrolidone - 69%
2-Phenylethyl methacrylate - 29%
allyl methacrylate (a crosslinker) - 1%
Lucirin TPO* (an initiator) - 1%
*diphenyl (2,4,6-trimethylbenzoyl) phosphine oxide
M1 made according to this formulation has a refractive index of between 1.414 and 1.416, a water content of between 58% to 60% and a swell factor of 1.34.

Coating 16 is used to cover diffractive surface 18 of base lens 14 and to provide a smooth surface so as to prevent any cellular ingrowth and resulting opacification along diffractive surface 18. So as to reduce the overall thickness of ICL 10, coating 16 preferably is less than 20 microns thick. The material used to make coating 16 (M2) preferably has a refractive index close to that of the comeal tissue and an equilibrium water content at approximately body temperature of at least 70%. M2 must be bondable to M1 with similar swelling properties so as to not delaminate. M2 should not distort or craze during rolling or folding of ICL 10, and preferably should cure rapidly (*e*.*g*., in less than 3 minutes). M2 must be photo stable and preferably already has been shown safe in the contact lens and/or intraocular lens industry.
One preferred formulation for M2 is:
polyvinylpyrrolidone (MW - 10K) (a plasticizer) - 19%
polyethylene glycol 200 ( a plasticizer) - 29%
glyceryl methacrylate - 49%
ethyleneglycol dimethacrylate ( a crosslinker)- 0.5%
Darocur 1173* (an initiator) - 2.5%
*2-hydroxy-2-methyl-1-phenyl-propan-1-one
M2 made according to this formulation has a refractive index of 1.376, a water content of approximately 73% and a swell factor of 1.30.

ICL 10 is made using conventional molding techniques well-known in the contact lens and intraocular lens art. See, for example, U.S. Patent No. 5,620,720 (Click, et al.)

A flexible bottom mold made from, for example, polypropylene, is filled with material M1. A first top mold made from, for example, fluoroethylene polypropylene (FEP), and containing the lens base curve and diffractive surface 18 is placed over the M1 containing bottom mold. M1 is cured, for example, under blue light (450 nm) at a flux of 14-15 mW/cm² for one hour. Alternatively, M1 can be cured by replacing Lucirin TPO with 1% t-butylperoxy(2-ethyl-hexanoate) thermal initiator and thermal curing at 80°C for 1 hour followed by a post-cure period of 1 hour at 100°C. The first top mold is removed and material M2 is placed on diffractive surface 18 of the newly formed base lens 14. A second top mold, also made from FEP and having the same base curve as the first top mold but with no diffractive surface 18 is placed over the bottom mold. Pressure is applied to the top mold at 689 kPa (approximately 100 lbs./in²) and the mold assembly is exposed to ultraviolet light (366 nm) at a flux of 60-300 mW/cm² for three minutes. The second top mold is then removed and ICL 10 along with the bottom mold is placed in 65-75°C heptane for several hours to extract the non-polymerized monomers. ICL 10 is removed from the bottom mold, allowed to air dry for several minutes and hydrated for at least two hours in hot, distilled water.

This description is given for purposes of illustration and explanation. It will be apparent to those skilled in the relevant art that changes and modifications may be made to the invention described above without departing from its scope.

## Claims

1. An intracomeal diffractive lens (10), comprising;
(a) a base lens (14) made from a first hydrogel material (M1) and having a refractive index greater than 1.40;
(b) a diffractive surface (18) formed on the base lens(14); and
(c) a coating (16) covering the diffractive surface, the coating made from a second hydrogel material (M2) having a refractive index that is less than 1.40,
**characterized in that** the first hydrogel material (M1) comprises N-vinyl-pyrrolidone and 2-Phenylethyl methacrylate, and the second hydrogel material (M2) comprises glyceryl methacrylate.

2. The intracorneal diffractive lens of claim 1, wherein the second material (M2) has a refractive index of approximately 1.37.

3. The intracorneal diffractive lens of claim 1 or claim 2, wherein the first material (M1) has a water content of at least 50%.

4. The intracorneal diffractive lens of claim 1 or claim 2, wherein the second material (M2) has a water content of at least 70%.

## Patentansprüche

1. Intracorneale lichtbrechende Linse (10), welche umfaßt:
(a) eine Basislinse (14), die aus einem ersten Hydrogelmaterial (M1) hergestellt ist und einen Brechungsindex von mehr als 1,4 aufweist,
(b) eine lichtbrechende Oberfläche (18), welche auf der Basislinse (14) ausgebildet ist, und
(c) eine Beschichtung (16), welche die lichtbrechende Oberfläche bedeckt, wobei die Beschichtung aus einem zweiten Hydrogelmaterial (M2) mit einem Brechungsindex von weniger als 1,4 hergestellt ist,
**dadurch gekennzeichnet, daß** das erste Hydrogelmaterial (M1) N-Vinylpyrrolidon und 2-Phenylethylmethacrylat und das zweite Hydrogelmaterial (M2) Glyzerylmethacrylat aufweist.

2. Intracorneale lichtbrechende Linse nach Anspruch 1, bei welcher das zweite Material (M2) einen Brechungsindex von ungefähr 1,37 aufweist.

3. Intracorneale lichtbrechende Linse nach Anspruch 1 oder Anspruch 2, bei welcher das erste Material (M1) einen Wassergehalt von mindestens 50% aufweist.

4. Intracorneale lichtbrechende Linse nach Anspruch 1 oder nach Anspruch 2, bei welcher das zweite Material (M2) einen Wassergehalt von mindestens 70% aufweist.

## Revendications

1. Lentille diffractive intra-cornéenne (10), comportant ;
(a) une lentille de base (14) constituée d'un premier matériau d'hydrogel (M1) et ayant un indice de réfraction supérieur à 1,40,
(b) une surface diffractive (18) formée sur la lentille de base (14), et
(c) un revêtement (16) recouvrant la surface diffractive, le revêtement étant constitué d'un second matériau d'hydrogel (M2) ayant un indice de réfraction qui est inférieur à 1,40,
**caractérisée en ce que** le premier matériau d'hydrogel (M1) comporte du N-vinylpyrrolidone et du méthacrylate de 2-Phényléthyle, et le second matériau d'hydrogel (M2) comporte du méthacrylate de glycéryle.

2. Lentille diffractive intra-cornéenne selon la revendication 1, dans laquelle le second matériau (M2) a un indice de réfraction d'approximativement 1,37.

3. Lentille diffractive intra-cornéenne selon la revendication 1 ou 2, dans laquelle le premier matériau (M1) a une teneur en eau d'au moins 50 %.

4. Lentille diffractive intra-cornéenne selon la revendication 1 ou 2, dans laquelle le second matériau (M2) a une teneur en eau d'au moins 70 %.
